# EUROPÄISCHE PATENTSCHRIFT

(11) **EP 3 975 965 B1**
(45) Veröffentlichungstag und Bekanntmachung des Hinweises auf die Patenterteilung: **19.08.2026**
(21) Anmeldenummer: 20730180.5
(22) Anmeldetag: 25.05.2020
(51) Int. Cl.: A61G 1/04, A61F 7/00

(54) **PERSONEN-RETTUNGSHILFSSYSTEM**
PERSON RESCUE SUPPORTING SYSTEM
SYSTÈME D'AIDE AU SAUVETAGE DE PERSONNES

(30) Priorität: 28.05.2019 DE 102019114299
(43) Veröffentlichungstag der Anmeldung: 06.04.2022
(73) Patentinhaber: Kleen, Stefan, 23816 Neversdorf (DE)
(72) Erfinder: Kleen, Stefan, 23816 Neversdorf (DE)
(74) Vertreter: patcare Patentanwälte Partnerschaft mbB
(86) Internationale Anmeldenummer: PCT/DE2020/100442
(87) Internationale Veröffentlichungsnummer: WO 2020/239173

(56) Entgegenhaltungen:
- DE-U1- 202019 103 009
- US-A- 6 113 626
- US-B2- 6 855 158

## Beschreibung

Die Erfindung betrifft ein Personen-Rettungshilfssystem für Rettungsdienste insbesondere zur Versorgung präklinischer Notfallpatienten.

Bisher finden im bodengebundenen Rettungsdienst aktive Wärmeerhaltungssysteme keine Verwendung. Wärmerhalt wird derzeit ausschließlich passiv mittels Rettungsdecken und eines aufgewärmten Rettungswagens (RTW, NKTW, ITW oder mobile intensice care unit) sowie für Trauma und schwerstverletzte Patienten invasiv mittels vorgewärmter Infusionen durchgeführt. Bei Letzteren handelt es sich um schwer kontrollierbare Wärmezufuhr zumal Infusionen auch nur in begrenzter Anzahl verabreicht werden können. Dies ist eine deutliche Lücke in der derzeitigen Rettungsdienst Praxis sowohl im Umgang mit Trauma Patienten als auch mit weiteren Notfallpatienten und bei Krankentransporten. Bei der Luftrettung und an Bord eines Rettungsschiffes erfolgen diese Maßnahmen soweit möglich analog.

Aus dem **Stand der Technik** sind unterschiedliche Rettungshilfssysteme für Rettungsdienste bekannt.

Aus der Druckschrift DE 10 2008 044 388 A1 sind Mehrphasenmaterialien bekannt, deren mechanische Eigenschaften durch von außen einwirkende elektrische oder magnetische Kräfte bzw. Felder verändert werden können. Insbesondere betrifft diese Erfindung Materialien aus mindestens zwei Phasen, die mit jeweils mindestens einem funktionalen Nanopartikel modifiziert sind und durch die unterschiedliche elektrische bzw. magnetische Reaktion der Nanopartikel in Verbindung mit den speziellen Eigenschaften der Matrix zu reversibel veränderlichen mechanischen Eigenschaften führen.

Die WO 2008/116847 A2 beschreibt ein Material zur Formung von Strukturen, bestehend aus einem Polymer oder einem Polymergemisch und einem Initiator, wobei das Polymer oder das Polymergemisch durch den Initiator von einem flüssigen oder viskosen Zustand in einen festen Zustand überführbar ist und dessen Verwendung sowie eine Verfahren zur Herstellung von Strukturen mit diesem Material.

Aus der Druckschrift US 6,912,747 B2 ist ein umhüllender Patiententräger bekannt, welcher eine flexible Oberfläche aufweist, die mit einer flexiblen Unterseite verbunden ist. Die flexible obere Oberfläche definiert eine wiederverschließbare Torsoöffnung, eine Gesichtsöffnung und eine Vielzahl von wiederverschließbaren Öffnungen für die medizinische Versorgung.

Weiter ist aus der Druckschrift US 2013/0041441 A1 eine Hypothermiemanagementvorrichtung, umfassend, einen Aufnahmehohlraum, der durch eine äußere Hülle definiert ist, die so angeordnet und verschließbar ist, dass diese eine verletzte Person einschließt; eine innere Schicht, die an der Außenhülle angebracht ist, wobei die innere Schicht eine untere Lage umfasst, die im Allgemeinen unter der verletzten Person angeordnet ist, wenn die verletzte Person in den Aufnahmehohlraum gebracht wird und eine obere Lage, die im Allgemeinen über der verletzten Person angeordnet ist, wenn die verletzte Person im aufnehmenden Hohlraum platziert wird; eine innere Auskleidung, die an der unteren Lage der inneren Schicht angebracht ist und einen inneren Hohlraum bildet; und eine Absorptionsschicht, die an der Innenauskleidung befestigt ist und im Allgemeinen unter der verletzten Person angeordnet ist, wenn die verletzte Person in der Außenhülle platziert ist und eine Heizanordnung, die in dem inneren Hohlraum angeordnet ist, bekannt.

Die DE 102 28 015 A1 beschreibt eine Patientenlagerungsmatte und deren Verwendung. Eine derartige Patientenlagerungsmatte weist eine flexible, luftundurchlässige Hülle auf, deren Innenraum ein partikuläres Füllmaterial enthält, und ein Ventil, über das der Innenraum be- und entlüftbar ist. Erfindungsgemäß ist zusätzlich ein flächiges, flexibles Heizelement vorgesehen, so dass die Patientenlagerungsmatte an die Körperkonturen eines Patienten anpassbar ist. Das kommt Teilen der vorliegenden Erfindung recht nahe. Allerdings handelt es sich hierbei nur um eine Matte als Unterlage und dient somit nicht zum Ruhigstellen des gesamten Körpers.

Die Druckschrift US 6,113,626 A umfasst eine Wärmeübertragungsdecke, die den Rumpf und die Beine umhüllt und die Arme, das Gesäß, das Perineum und den Kopf freilässt und die selektive Erwärmung oder Abkühlung verschiedener Körperteile mit gleicher oder unterschiedlicher Geschwindigkeit ermöglicht. Die Decke der vorliegenden Erfindung besteht auch aus Paneelen, die selektiv geöffnet werden können, um Zugang zur Brust, zum Bauch, zu den Beinen oder zum Rücken zu erhalten, um ein Operationsfeld freizulegen oder um Zugang zu diesen Bereichen für die notwendige medizinische Versorgung zu ermöglichen.

Zudem offenbart die Druckschrift US 6 855 158 B2 eine Vorrichtung zur Regelung der Temperatur einer Körperstützstruktur mit mindestens einer Blase. Ein Temperaturmodulator ist mit der mindestens einen Blase verbunden, um die Temperatur der Flüssigkeit in der Blase zu verändern.

Inwieweit die Verwendung von bestimmten Materialien, wie z.B. in der DE 10 2008 044 388 A1 oder WO 2008/116847 A2 beschrieben, zu diesem Zeitpunkt bereits relevant sind, kann im Moment nicht eingeschätzt werden. Dies wird sich erst in der Entwicklung zeigen. Ebenso verhält es sich mit beschriebenen Funktionsweisen wie z.B. den unterschiedlichen Lösungen für Wärmedecken oder wärmeleitfähigen Materialien.

Die DIN EN 1789 Typ C regelt die Ausstattung mit Multifunktionswerkzeugen einer "mobile intensive care unit" für den Einsatz der "Rettungsdienstfahrzeuge und deren Ausrüstung - Krankenkraftwagen", bekannt als Rettungs- oder Notarztwagen, u.a. mit einer Fahrtrage, Schaufeltrage, Vakuummatraze.

Die **Probleme im Stand der Technik** sind im Wesentlichen, dass aus Mangel an Möglichkeiten ein kontrolliertes Temperaturmanagement derzeit erst im Krankenhaus (niederschwellig durch vorgewärmte Decken oder die o.g. Patientenlagerungsmatte etc. bis hin zur aktiven Erwärmung durch eine Herzlungenmaschine) beginnt. Dabei kann ein präklinisches kontrolliertes Temperaturmanagement für Patienten über lebenswichtig sein. Beispielsweise kann eine Abweichung der Körpertemperatur von bereits 1° C unter Normtemperatur mit einem Einbruch von bis zu 20 % der Blutgerinnung einhergehen. Dies bedeutet insbesondere bei schwerst- oder Polytraumatisierten Patienten ein schlechteres Langzeitüberleben.

Es wurde insbesondere erkannt, dass bei im Stand der Technik befindlichen Ausrüstungsmitteln für ein Rettungsfahrzeug eine Temperierung des Patienten nur zusammen mit den Rettern durch die Heizung oder Klimaanlage des Fahrzeugs erfolgen kann. Je nach Witterung kann dies zur erheblichen Behinderung der Rettungskräfte in der Versorgung des Patienten führen.

Der vorliegenden Erfindung liegt die **Aufgabe** zugrunde, ein Personen-Rettungshilfssystem als präklinisches Temperatur und Trauma Management Systems zu entwickeln. Dieses soll zur Verbesserung der Langzeitüberlebenschancen bei Trauma- und Notfallpatienten sowie die Erhöhung des Komforts bei Notfall- und Krankentransporten dienen.

**Gelöst** wird diese Aufgabe mit einem Personen-Rettungshilfssystem für verletzte Personen gemäß Hauptanspruch.

Das Personen-Rettungshilfssystem für verletzte Personen weist wenigstens zwei Module mit Kühl- und/oder Heizmitteln auf, wobei diese wenigstens teilweise verwendungsgemäß den Körper oder Körperabschnitte einer Person einhüllend bedeckend ausgebildet sind und wobei diese Module einzeln und unabhängig von dem oder den weiteren Modulen in Bezug auf Temperatur thermisch variabel regelbar und/oder ansteuerbar sind, dadurch gekennzeichnet, dass
- zur thermischen Aktivierung und Ansteuerung der thermisch aktivierbaren Module eine Regelungs- / Interfaceeinheit und/oder zentrale Steuereinheit vorgesehen ist;
- Temperatursensoren in den Modulen vorgesehen sind, deren Messwerte zur thermischen Aktivierung und Ansteuerung der thermisch aktivierbaren Module über die Regelungs- / Interfaceeinheit und/oder zentrale Steuereinheit genutzt werden;
- die Temperaturregelung der Module lokal regelbar direkt körpernah erfolgt; und
- die thermisch aktivierbaren Module als elektrische Heizung/Kühlung und/oder als Peltierelemente und/oder als Heiz-/Kühlelemente auf Basis chemischer, biologischer Energiewandlungsprozesse ausgebildet sind.

Besonders bevorzugt können die Module die Extremitäten oder Abschnitte der Extremitäten einer Person und/oder den Rumpf einer Person und/oder den Kopf einer Person wenigstens teilweise einhüllend ausgebildet sein.

Die einzelnen Module können miteinander über Klettverbindungen und/oder Reißverschlüsse und/oder Knopfverbindungen und/oder Verbindungen und/oder Verschlüsse verbindbar sein. Die Module entlang der Längserstreckung können eine über Klettverbindungen und/oder Reißverschlüsse und/oder Knopfverbindungen und/oder Verbindungen und/oder Verschlüsse öffenbare und verschließbare Öffnung zum Anlegen an den und Abnehmen von dem jeweiligen Körperabschnitt aufweisen.

Ferner können die Module als eine durchgängige Einhüllende für die verletzte Person zusammenfassbar sein.

In einer weiteren Ausgestaltung kann eine Unterlage vorgesehen sein, wobei die Unterlage als gemeinsames Unterteil der Module ausgebildet sein kann, wobei innerhalb der Unterlage mehrere Zonen vorgesehen sein können, die thermisch einzeln und individuell ansteuerbar und/oder regelbar sein können, wobei ein Kühlen und/oder ein Wärmen der einzelnen Bereiche möglich ist.

Ferner kann erfindungsgemäß eine Polsterliege vorgesehen sein.

In einer weiteren zusätzlichen Variante kann die Polsterliege mit Fixiermitteln für den Transport und/oder die Stabilisierung ausgebildet sein.

Eine weitere Lösung für die oben beschriebene Problematik kann ein Personen-Rettungshilfssystem in der Anwendung als ein "Temperatur reguliertes Patienten Transport- und Trauma Management System" sein.

Eine weitere Anwendung ist für Trauma Patienten (inklusive Immobilisation) für Krankentransporte (z.B. ITW Intensivtransportwagen) gegeben.

Durch die Modularität und Flexibilität im Aufbau ist das erfinderische Personen-Rettungshilfssystem nicht eingeschränkt und kann für eine Vielzahl von Rettungssituationen bei Notfallpatienten und/oder Krankentransporten eingesetzt werden und deckt somit das komplette Einsatzspektrum im Luft- und Bodengebundenen Rettungsdienst sowie der Bergrettung ab. Die Möglichkeit der Temperaturregulierung erfolgt bevorzugt über ein beheizbares Textilinlet welches die Energieversorgung des Rettungsfahrzeugs und/oder eine autarke Energieversorgung, z.B. über Stromaggregate oder Akkumulatoren nutzt. Die Art der Energieversorgung richtet sich dabei nach Einsatzort und Versorgungsmöglichkeit. Für einen Rettungs-/Bergesack, wie er derzeit in der Luft- und Bergrettung genutzt wird, bietet sich der Einsatz von Akkumulatoren als Energieversorgung an. Gleichzeitig wird in dem Bergesack zur Immobilisation von Trauma Patienten eine Vakuummatratze integriert. Zur Anwendung kommen dabei moderne Materialien, wie z.B. Carbon für eine Fußplatte sowie Längsverstrebungen, die die Steifigkeit verbessern ohne das Gewicht wesentlich zu erhöhen. Die Oberflächen sowie auch das Inlet sind bevorzugt aus leicht zu reinigenden bzw. zu desinfizieren Materialien, wie z.B. Complan^{®}, gefertigt.

Die Vorteile des erfinderischen Personen-Rettungshilfssystems

Das als ein Rettungssack geformte mehrteilige Verschlusssystem 2 kann bereits im Haus angelegt werden, wodurch ein zweites Umlagern des Patienten bzw. die Nutzung von Rettungstuch, Schaufeltrage sowie Spineboard zum Transfer durch Treppenhäuser und die damit verbundenen Risiken entfallen.

Bei Traumapatienten kann bereits vor dem Eintreffen im Krankenhaus einer Hypothermie kontrolliert entgegengewirkt werden. Dadurch geht keine wertvolle Zeit verloren.

Die kontrollierte Wärmezufuhr und die dadurch wärmere Körpertemperatur des Patienten führt zu einer Verbesserung physiologischer Eigenschaften, beispielsweise eine bessere Wirkung von Medikamenten oder die Stabilisierung der Gerinnung. Gleichzeitig wird auch hier die Hypothermie bekämpft. Dies kann insbesondere für Notfallpatienten entscheidend sein.

Die Besatzung des RTWs muss nicht mehr in einem deutlich überhitzen oder unterkühlten RTW arbeiten, um Patienten unkontrollierbar zu temperieren.

Diese Temperierung erfolgt lokal regelbar direkt körpernah am Patienten durch das als Rettungssack geformte mehrteilige Verschlusssystem 2.

Der Komfort für den Patienten erhöht sich durch diese direkte lokale Temperaturregelung im Rettungssack, weil durch das direkt am Körper des Patienten anliegende System die Wärme- oder Kältezufuhr am bedürftigen Körperteil deutlich besser reguliert werden, als über die Luft im Innenraum durch die Klimaanlage des RTW's.

Durch die integrierte Temperatur Steuerung oder Regelung des Systems lässt sich eine Zieltemperatur und eine Zeit bis diese erreicht wird genau definieren. Dadurch kann ein sogenannter "after drop" durch eine zu schnelle Erwärmung der Extremitäten vermieden werden.

Insgesamt verbessert das erfinderische Personen-Rettungshilfssystem, z.B. durch ein "Temperatur reguliertes Patienten Transport- und Trauma Management System" die Langzeitüberlebenschancen bei Trauma und Notfall Patienten. Es führt zu einer deutlichen Erhöhung des Patienten Komforts bei Notfall- und Krankentransporten und nicht zuletzt auch zu einer Verbesserung des Komforts der RTW Besatzung.

Nachfolgend werden Ausführungsbeispiele der Erfindung anhand der beiliegenden Zeichnungen in der **Figurenbeschreibung** detailliert beschrieben, wobei diese die Erfindung erläutern sollen und nicht beschränkend zu werten sind:
Es zeigen:
- Fig. 1: ein Personen-Rettungshilfssystem in Aufsicht als schematische Darstellung eines ersten Ausführungsbeispiels;
- Fig. 2: ein Personen-Rettungshilfssystem in Aufsicht nach Figur 1 als schematische Darstellung eines Ausführungsbeispiels im Zustand der partiellen Aktivierung und qualitativen Regelung der Wärmefunktion oder Kühlfunktion;
- Fig. 3: ein Personen-Rettungshilfssystem in Aufsicht nach Figur 1 als schematische Darstellung eines Ausführungsbeispiels wobei einzelne Teile des mehrteiligen Verschlusssystems zu Einheiten miteinander verbunden sind und einen Rettungssack bilden;
- Fig. 4: ein Personen-Rettungshilfssystem in Aufsicht nach Figur 1 als schematische Darstellung eines Ausführungsbeispiels im Zustand der vollständigen Aktivierung der Wärmefunktion;
- Fig. 5: ein Personen-Rettungshilfssystem in Aufsicht nach Figur 1 als schematische Darstellung eines Ausführungsbeispiels im Zustand der vollständigen Aktivierung der Kühlfunktion;
- Fig. 6: ein Personen-Rettungshilfssystem in Aufsicht nach Figur 1 mit externer Regelungs-/Interfaceeinheit
- Fig. 7: mehrere Personen-Rettungshilfssysteme in Aufsicht nach Figur 1 mit einer externen Regelungs-/Interfaceeinheit
- Fig. 8: Beispiel eines Personen-Rettungshilfssystems in Explosionsansicht;
- Fig. 9: Beispiel eines offenen Personen-Rettungshilfssystems und
- Fig. 10: Beispiel eines geschlossenen Personen-Rettungshilfssystems.

An dieser Stelle soll darauf hingewiesen werden, dass der Gegenstand der Erfindung nicht auf die Beispiele beschränkt ist.

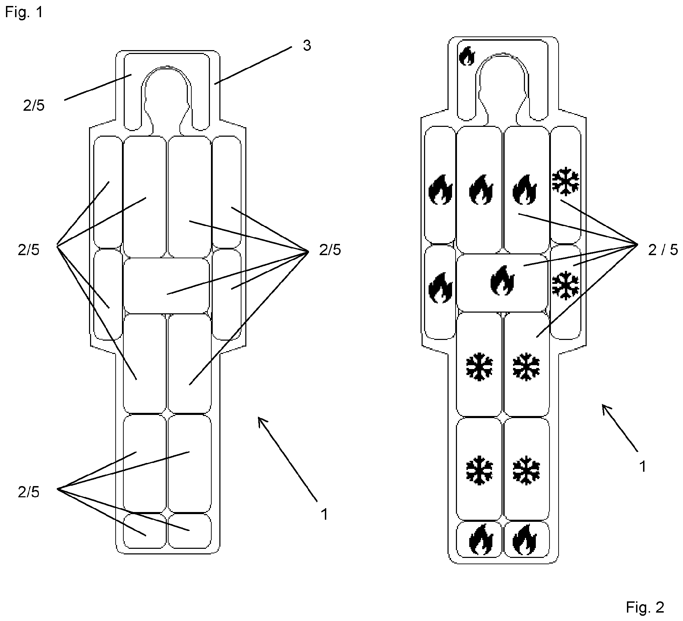

In **Fig. 1** ist eine Personen-Rettungshilfssystem 1 in Aufsicht als schematische Darstellung eines ersten Ausführungsbeispiels mit einem mehrteiligen Verschlusssystem 2 / Modulsystem 5, welches eine angedeutete Person (Kopf) umfasst dargestellt. Das mehrteilige Verschlusssystem 2 / Module 5 ist in Aufsicht gezeigt, umfasst die Person vollständig und liegt mit dieser auf einer Polsterliege 3 / Modul 5 und bildet für die Person einen Rettungssack, bestehend aus mehreren funktionalen Teilen mit den thermisch aktivierbare Modulen 5. Im Beispiel bildet eines der mehrteiligen Verschlusssysteme 2 / Module 5 eine funktionale Kopfumfassung, die sowohl den Kopf, Hinterkopf und die Halsregion umfassen und stabilisieren kann. Alle Teile des mehrteiligen Verschlusssytems 2 / Module 5 können miteinander gekoppelt werden, so dass sie einen Rettungssack bilden, der ähnlich wie ein Schlafsack mit Kopfteil aufgebaut ist. Zusätzlich kann jedes Teil auch einzeln entfernt werden, so dass ein Retter ein Körperteil freilegen kann, z.B. um direkt Zugang zu einem zu versorgenden Körperteil zu erlangen, ohne die anderen Körperteile aufzudecken.

**Fig. 2** zeigt das Personen-Rettungshilfssystem 1 in Aufsicht nach Figur 1 als schematische Darstellung eines Ausführungsbeispiels im Zustand der partiellen Aktivierung und qualitativen Regelung der Wärmefunktion und bzw. oder Kühlfunktion über die jeweiligen thermisch aktivierbaren Module 5.

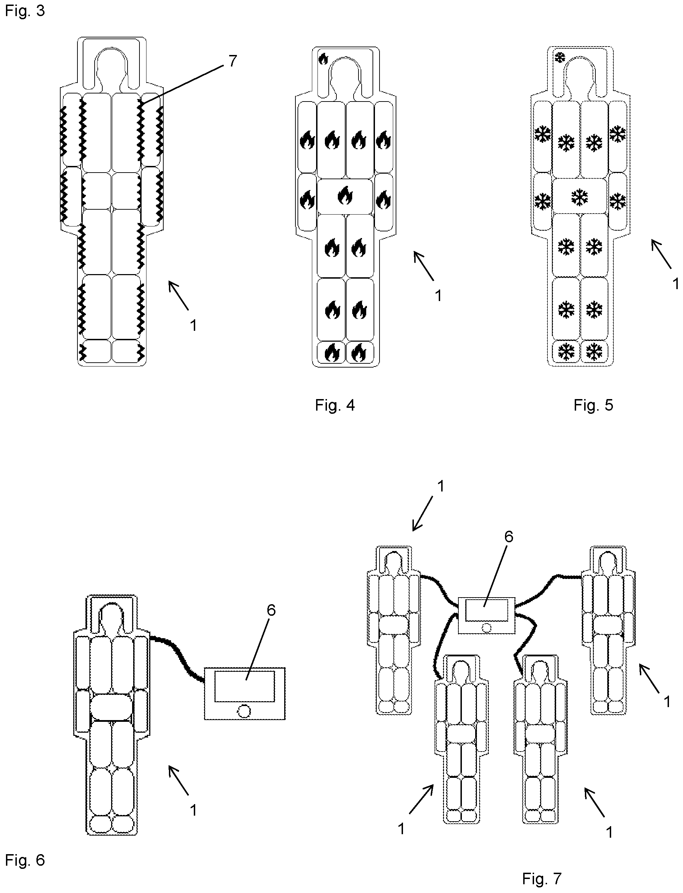

**Fig. 3** zeigt das Personen-Rettungshilfssystem 1 in Aufsicht nach Figur 1 als schematische Darstellung eines Ausführungsbeispiels wobei einzelne Teile des mehrteiligen Verschlusssystems 2 / Module 5 zu Einheiten miteinander jeweils über einen Verschluss 7 verbunden sind und einen Rettungssack bilden, der entlang der Körperlängsachse zwischen Hals und Füßen eine freilegbare aufschlagbare Öffnung bereitstellt über die ein Retter Zugriff auf die Körpermitte erhält.

In **Fig. 4** ist das Personen-Rettungshilfssystem 1 in Aufsicht nach Figur 1 als schematische Darstellung eines Ausführungsbeispiels im Zustand der vollständigen Aktivierung der Wärmefunktion für alle Teile des mehrteiligen Verschlusssystems 2 / Module 5 gezeigt. Dabei deuten die Flammensymbole die relative Qualität der Wärmezufuhr an. So ist z.B. der Kopfbereich schwächer erwärmt als die restlichen Teile des mehrteiligen Verschlusssystems 2.

**Fig. 5** ist das Personen-Rettungshilfssystem 1 in Aufsicht nach Figur 1 als schematische Darstellung eines Ausführungsbeispiels im Zustand der vollständigen Aktivierung der Kühlfunktion für alle Teile des mehrteiligen Verschlusssystems 2 gezeigt. Dabei deuten die Schneeflockensymbole die relative Qualität der Kältezufuhr an. So ist z.B. der Kopfbereich schwächer gekühlt als die restlichen Teile des mehrteiligen Verschlusssystems 2.

**Fig. 6** zeigt das Personen-Rettungshilfssystem 1 in Aufsicht nach Figur 1 mit externer Regelungs-/Interfaceeinheit 6 welches die einzelnen thermisch aktivierbaren Module 5 ansteuert oder regelt und mit einer Energieversorgung verbindet. Das Regelungs- / Interfaceeinheit 6 kann über eine eigene Informations- und Protokoll-/Dokumentationseinheit die Retter mit Assistenzsystemen unterstützen.

**Fig. 7** zeigt mehrere Personen-Rettungshilfssystem 1 in Aufsicht nach Figur 1 mit einer externen Regelungs- / Interfaceeinheit 6 welches die einzelnen thermisch aktivierbaren Module 5 des jeweiligen Personen-Rettungshilfssystems 1 ansteuert oder regelt und mit einer Energieversorgung verbindet. Das Regelungs- / Interfaceeinheit 6 kann über eine eigene Informations- und Protokoll-/Dokumentationseinheit die Retter mit Assistenzsystemen unterstützen.

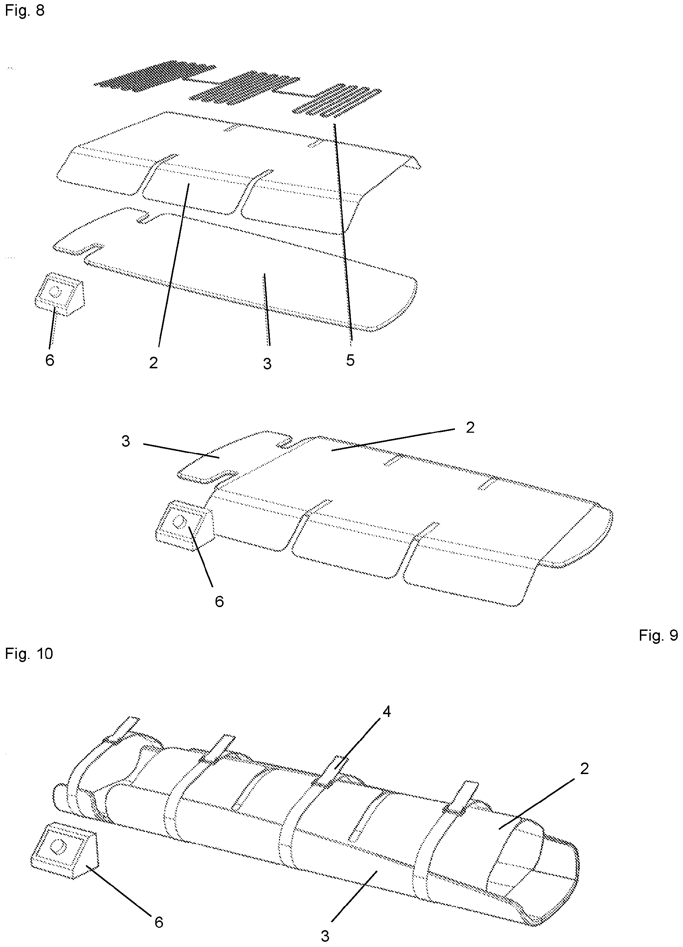

**Fig. 8** zeigt ein beispielhaftes Personen-Rettungshilfssystem 1 in Explosionsansicht. Auf der Polsterliege 3 liegt ein mehrteiliges Verschlussystem 2, hier mit ausgelassenen Bereichen und als Faltzonen angedeuteten Bereichen, die eine jeweilige einfache zonale Faltung und Bewegung von Teilbereichen des mehrteiligen Verschlusssystems 2 ermöglichen. Die Polsterliege ist eine gepolsterte Auflage für einen Körper, bevorzugt als Vakuumpolster ausgeführt. Die thermisch aktivierbaren Module 5, hier in der Figur 1 als eine elektrische Heizeinheit gezeigt, können als additive zonale Einheiten oder als in das mehrteilige Verschlusssystem und/oder die Polsterliege integrierte Einheiten ausgeführt werden. Die thermisch aktivierbaren Module 5 sind als Kühl- und/oder Heizeinheiten ausgeführt, z.B. als elektrische Heizung/Kühlung, als Peltierelemente, als Heiz-/Kühlelemente auf Basis chemischer, biologischer Energiewandlungsprozesse. Die Geometrie der Polsterliege 3 ist an gängige Körpergeometrien hinsichtlich Länge und Breite anpassbar. Durch die Mehrteiligkeit des Verschlussystems 2 ist eine zonale Bedeckung oder Freilegung eines Körpers möglich.

**Fig. 9** zeigt ein Beispiel eines offenen Personen-Rettungshilfssystems 1 nach Figur 8.

**Fig. 10** zeigt ein Beispiel eines geschlossenen Personen-Rettungshilfssystems 1 nach Figur 9 mit ergänzenden Fixiermitteln 4, wie z.B. Gurtbändern.

### Bezugszeichenliste

- 1: Personen-Rettungshilfssystem
- 2: Mehrteiliges Verschlusssystem
- 3: Polsterliege
- 4: Fixiermittel
- 5: Thermisch aktivierbare Module
- 6: Regelungs-/Interfaceeinheit
- 7: Verschluss

## Patentansprüche

1. Personen-Rettungshilfssystem (1) für verletzte Personen aufweisend wenigstens zwei Module (5) mit Kühl- und/oder Heizmitteln, wobei diese wenigstens teilweise verwendungsgemäß den Körper oder Körperabschnitte einer Person einhüllend bedeckend ausgebildet sind und wobei diese Module (5) einzeln und unabhängig von dem oder den weiteren Modulen (5) in Bezug auf Temperatur thermisch variabel regelbar und/oder ansteuerbar sind,
**dadurch gekennzeichnet, dass**
- zur thermischen Aktivierung und Ansteuerung der thermisch aktivierbaren Module (5) eine Regelungs- / Interfaceeinheit (6) und/oder zentrale Steuereinheit vorgesehen ist;
- Temperatursensoren in den Modulen (5) vorgesehen sind, deren Messwerte zur thermischen Aktivierung und Ansteuerung der thermisch aktivierbaren Module (5) über die Regelungs- / Interfaceeinheit (6) und/oder zentrale Steuereinheit genutzt werden;
- die Temperaturregelung der Module (5) lokal regelbar direkt körpernah erfolgt; und
- die thermisch aktivierbaren Module (5) als elektrische Heizung/Kühlung und/oder als Peltierelemente und/oder als Heiz-/Kühlelemente auf Basis chemischer, biologischer Energiewandlungsprozesse ausgebildet sind.

2. Personen-Rettungshilfssystem (1) nach Anspruch 1,
**dadurch gekennzeichnet, dass**
die Module (5)
- die Extremitäten oder Abschnitte der Extremitäten einer Person
und/oder
- den Rumpf einer Person;
und/oder
- den Kopf einer Person wenigstens teilweise einhüllend ausgebildet sind.

3. Personen-Rettungshilfssystem (1) nach Anspruch 1 oder 2,
**dadurch gekennzeichnet, dass**
die einzelnen Module (5) miteinander über Klettverbindungen und/oder Reißverschlüsse und/oder Knopfverbindungen und/oder Verbindungen und/oder Verschlüsse (7) verbindbar sind.

4. Personen-Rettungshilfssystem (1) nach einem der vorangehenden Ansprüche
**dadurch gekennzeichnet, dass**
die Module (5) entlang der Längserstreckung eine über Klettverbindungen und/oder Reißverschlüsse und/oder Knopfverbindungen und/oder Verbindungen und/oder Verschlüsse (7) öffenbare und verschließbare Öffnung zum Anlegen an den und Abnehmen von dem jeweiligen Körperabschnitt aufweisen.

5. Personen-Rettungshilfssystem (1) nach einem der vorangehenden Ansprüche
**dadurch gekennzeichnet, dass**
die Module (5) als eine durchgängige Einhüllende für die verletzte Person zusammenfassbar sind.

6. Personen-Rettungshilfssystem (1) nach einem der vorangegangenen Ansprüche,
**dadurch gekennzeichnet, dass**
eine Unterlage vorgesehen ist, wobei die Unterlage als gemeinsames Unterteil der Module (5) ausgebildet ist, wobei innerhalb der Unterlage mehrere Zonen vorgesehen sind, die thermisch einzeln und individuell ansteuerbar und/oder regelbar sind, wobei ein Kühlen und/oder ein Wärmen der einzelnen Bereiche möglich ist.

7. Personen-Rettungshilfssystem (1) nach einem der vorangegangenen Ansprüche,
**dadurch gekennzeichnet, dass**
eine Polsterliege (3) vorgesehen ist.

8. Personen-Rettungshilfssystem (1) nach dem vorangegangenen Anspruch,
**dadurch gekennzeichnet, dass**
die Polsterliege (3) mit Fixiermitteln (4) für den Transport und/oder die Stabilisierung ausgebildet ist.

## Claims

1. Personal rescue aid system (1) for injured persons, comprising
at least two modules (5) with cooling and/or heating means, wherein these modules are configured, at least in part, to envelop and cover the body or parts of a person's body in accordance with the intended use, and wherein these modules (5) can be thermally controlled and/or driven individually and independently of the other module(s) (5) with regard to the temperature,
wherein
- a control-/interface unit (6) and/or a central control unit is provided for the thermal activation and control of the thermally activatable modules (5);
- temperature sensors are provided in the modules (5), their measured values being used for the thermal activation and control of the thermally activatable modules (5) via the control-/interface unit (6) and/or the central control unit;
- the temperature control of the modules (5) is carried out locally, directly close to the body;
and
- the thermally activatable modules (5) are designed as electric heating/cooling elements and/or as Peltier elements and/or as heating/cooling elements based on chemical, biological energy conversion processes.

2. Personal rescue aid system (1) according to claim 1,
wherein
the modules (5) are configured to enclose
- a person's limbs or sections of their limbs
and/or
- a person's torso;
and/or
- a person's head, at least partially.

3. Personal rescue aid system (1) according to claim 1 or 2,
wherein
the individual modules (5) can be connected to one another via Velcro fastenings and/or zips and/or button fastenings and/or connectors and/or fasteners (7).

4. Personal rescue aid system (1) according to one of the preceding claims
wherein
the modules (5) comprise, along their longitudinal extent, an opening which can be opened and closed by means of Velcro fastenings and/or zips and/or button fastenings and/or other fastenings (7) for applying to and removing from the respective body part.

5. Personal rescue aid system (1) according to one of the preceding claims
wherein
the modules (5) can be combined to form a continuous enclosure for the injured person.

6. Personal rescue aid system (1) according to one of the preceding claims,
wherein
a base is provided, wherein the base is configured as a common lower section of the modules (5), wherein several zones are provided within the base which can be thermally controlled and/or regulated individually and independently, whereby cooling and/or heating of the individual areas is possible.

7. Personal rescue aid system (1) according to one of the preceding claims,
wherein
padded couch (3) is provided.

8. Personal rescue aid system (1) according to the preceding claim,
wherein
the padded couch (3) is designed with securing means (4) for transport and/or stabilisation.

## Revendications

1. Système d'aide au sauvetage de personnes (1) destiné aux personnes blessées, comprenant au moins deux modules (5) dotés de moyens de refroidissement et/ou de chauffage, dans laquelle ces modules sont conçus pour envelopper et recouvrir, au moins en partie et conformément à leur utilisation, le corps ou des parties du corps d'une personne, et ces modules (5) pouvant être régulés et/ou commandés individuellement et indépendamment du ou des autres modules (5) en termes de température,
**caractérisé en ce que**
- une unité de régulation / d'interface (6) et/ou une unité de commande centrale est prévue pour l'activation thermique et la commande des modules (5) activables thermiquement ;
- des capteurs de température sont prévus dans les modules (5), dont les valeurs mesurées sont utilisées pour l'activation thermique et la commande des modules thermiquement activables (5) via l'unité de régulation/d'interface (6) et/ou l'unité de commande centrale ;
- la régulation de la température des modules (5) s'effectue localement, à proximité immédiate du corps
et
- les modules (5) activables thermiquement sont conçus sous forme de dispositifs électriques de chauffage/refroidissement et/ou d'éléments Peltier et/ou d'éléments de chauffage/refroidissement basés sur des processus de conversion d'énergie chimiques, biologiques.

2. Système d'aide au sauvetage de personnes (1) selon la revendication 1,
**caractérisé en ce que**
les modules (5) enveloppés
- les extrémités ou des parties des extrémités d'une personne
et/ou
- le torse d'une personne;
et/ou
- la tête d'une personne, au moins partiellement.

3. Système d'aide au sauvetage de personnes (1) selon la revendication 1 ou 2,
**caractérisé en ce que**
les différents modules (5) peuvent être reliés entre eux par des connexions Velcro et/ou des connexions à glissière et/ou des boutons et/ou des attaches et/ou des fermetures (7).

4. Système d'aide au sauvetage de personnes (1) selon l'une des revendications précédentes,
**caractérisé en ce que**
les modules (5) présentent, le long de leur extension longitudinale, une ouverture pouvant être ouverte et fermée à l'aide de fermetures velcro et/ou de fermetures à glissière et/ou de boutons et/ou de connexions et/ou de fermetures (7) pour permettre la mise en place sur la partie du corps concernée et le retrait de celle-ci.

5. Système d'aide au sauvetage de personnes (1) selon l'une des revendications précédentes,
**caractérisé en ce que**
les modules (5) peuvent être assemblés pour former une enveloppe continue autour de la personne blessée.

6. Système d'aide au sauvetage de personnes (1) selon l'une des revendications précédentes,
**caractérisé en ce que**
un support est prévu, dans lequel sont prévues plusieurs zones, lesquelles peuvent être commandées et/ou régulées thermiquement de manière individuelle et séparée, ce qui permet de refroidir et/ou de réchauffer les différentes zones.

7. Système d'aide au sauvetage de personnes (1) selon l'une des revendications précédentes,
**caractérisé en ce que**
un lit rembourrée (3) est prévue.

8. Système d'aide au sauvetage de personnes (1) selon la revendication précédente,
**caractérisé en ce que**
le lit rembourré (3) est muni de moyens de fixation (4) destinés au transport et/ou à la stabilisation.
